(19)

Europäisches
Patentamt

European
Patent Office

Office européen
des brevets

(11)     **EP 1 741 058 B1**

(12)                    **EUROPEAN PATENT SPECIFICATION**

(45) Date of publication and mention
of the grant of the patent:
**27.06.2018   Bulletin 2018/26**

(51) Int Cl.:
*G06T 5/00* *(2006.01)*          *G06T 5/40* *(2006.01)*
*A61B 6/03* *(2006.01)*

(21) Application number: **05736467.1**

(86) International application number:
**PCT/SE2005/000611**

(22) Date of filing: **28.04.2005**

(87) International publication number:
**WO 2005/106791 (10.11.2005 Gazette 2005/45)**

(54) **METHOD AND SYSTEM FOR AUTOMATICALLY IMPROVING THE USABILITY OF A MEDICAL PICTURE**

METHODE UND SYSTEM ZUR AUTOMATISCHEN VERBESSERUNG DER VERWENDBARKEIT EINES MEDIZINISCHEN BILDES

PROCEDE ET SYSTEME D'AMELIORATION AUTOMATIQUE DE L'UTILITE D'UNE IMAGE MEDICALE

(84) Designated Contracting States:
**AT BE BG CH CY CZ DE DK EE ES FI FR GB GR HU IE IS IT LI LT LU MC NL PL PT RO SE SI SK TR**

(30) Priority:  **30.04.2004   SE 0401126**

(43) Date of publication of application:
**10.01.2007   Bulletin 2007/02**

(73) Proprietor: **ELEKTA INSTRUMENT AB**
**S-103 93 Stockholm (SE)**

(72) Inventor: **ERIKSSON, Andreas**
**S-113 40 Stockholm (SE)**

(74) Representative: **AWA Sweden AB**
**P.O. Box 11394**
**404 28 Göteborg (SE)**

(56) References cited:
**US-A- 4 105 922        US-A- 4 688 175**
**US-A- 4 922 915        US-A- 4 975 970**
**US-A- 5 046 118        US-A- 5 956 435**
**US-B1- 6 466 687**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The present invention relates to a method and a system for automatically improving the usability of a medical picture. Further, the invention relates to various uses of such a method.

Background of the invention

Medical pictures and images find their use in a vast amount of different medical methods and therapies. For example, it is known to use medical images when preparing and conducting neurosurgical treatment of tumors, vascular deformities, and similar malformations in the brain. For this type of application, it is e.g. common to use a computer program, such as the commercially available GammaPlan program, which allows two or three dimensional viewing of target volumes and dose distribution as well as storage of lamina images of the brain that are obtained by imaging techniques such as computed tomography (CT), magnetic resonance imaging (MRI) and angiographic imaging, and in which the choice of irradiation points could be effected manually. It is also known to used automatized tools, e.g. a surgical planning system, such as the Leksell SurgiPlan. However, medical pictures are used in many other applications as well.

Further, US 5 046 118 discloses a way of adjusting the dynamic range for input X-ray picture to the dynamic range for a medium where the picture is to be presented, a so-called tone scale transformation. Here, a background grey scale level $X_B$ is first determined, and subsequently, by means of this background level, an area of interest, ROI. A conditional entropy (posteriori entropy) is used for the determination of the background level.

Additionally, US 5 956 435 discloses an automatic image analysis method in which two images which have been recorded at different instants are analyzed and a transformation function is determined on the basis of the shifts of image sections, which transformation function describes the shift from one image to another. In order to enable exact determination of this shift, corresponding image sections are determined in the two images by histogram analysis.

However, a common problem in assessing useful information from medical pictures, such as CT and MR pictures, is that the intensity range normally is very wide. For example, the intensity range for a CT image is typically about 4000 Hounsfields, ranging from air to parts with very high density, such as bone and skeleton parts, whereas the soft tissue normally is depictured in a very narrow intensity range, typically of about 100 Hounsfield, due to a smaller variation in density between these parts. However, for many types of surgery and therapy, the major or sole interest lies in the distinction between various parts of the soft tissue, and this could be very difficult from most medical pictures.

Accordingly, in order to deduce any medically useful information regarding the soft tissue from such a picture, it is of utmost importance to correctly control the intensity parameters of the pictures, and especially brightness and contrast, in order to delimit the gray scale window of the picture to a useful range. At the same time, it is important to control the intensity parameters in such a way that useful data is not lost. However, such an adequate control of the intensity parameters is a difficult, tedious and time consuming task, and requires high skills of the operator. The quality and usefulness of each picture is very much dependent on the individual skills of the operator in charge, leading to very varying results depending on the operator in charge. Further, the risks of human errors is high, with resulting difficulties for the physicians, and in the end increased health hazards for the patient to be treated.

Further, the quality and usefulness of the result provided by different tools using medical images as input, such as the above-discussed Gamma plan and Leksell SurgiPlan, are much dependent on the quality of the image information, and of the ability to distinguish between various soft tissue parts.

Still further, an often used technique in assessing useful and medically relevant information from medical images is to combine pictures, so called image merging or co-registration. This could be used in order to combine inter alia different types of pictures, e.g. CT and MR pictures. However, in order to provide correct co-registrations, a similarity metric must be computed, which requires that adequate intensity parameters for the images are known. Accordingly, also in this application it is of utmost importance to provide a good intensity resolution of the image parts of interest, e.g. parts corresponding to depicted soft tissue.

The same requirements apply to many other types of medically related picture analysis and picture manipulation, such as segmentation and pattern recognition.

Accordingly, there is a need for automatically improving the usability of a medical pictures.

Summary of the invention

It is therefore an object of the present invention to provide a method, system and computer software for automatically improving the usability of medical pictures.

This object is achieved with a method, system and computer software according to the appended claims. The invention also relates to certain applications for use of this new method.

According to a first aspect of the invention, it relates to a method of automatically improving the usability of a medical picture, comprising the step: providing as an input a medical picture, comprising an array of intensity data; characterized by the additional steps: automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby providing a decreased entropy outside the sub-range; and providing the processed array of intensity data of said sub-range as the improved medical picture. With this method, the usability of medical pictures, such as CT and MR pictures, is automatically improved. By the increase of the entropy in at least certain parts of the picture, the intensity resolution and possibility to distinguish between different parts of e.g. depictured soft tissue are greatly improved. However, the picture data is still maintained intact throughout the process, making the method medically reliable. Further, the method does not require any specific knowledge about the picture beforehand, regarding e.g. what object it depicts, how the image was made etc. On the contrary, the method provides an automatic improvement in the medical usability regardless of the input image, and without any additional information of the picture.

Further, apart from the above-discussed advantages, the method could also be implemented and executed very rapidly and cost effectively.

Since the improvement of the picture is performed automatically, no particular skills are required from the operator. Further, the end result is at least relatively independent on the operator, whereby a very reliable and secure end result is provided, thus alleviating the risk for human errors, and incurring no medical hazards for the patient.

Still further, the improved medical pictures also makes methods using such pictures more effective and more reliable.

In this application, medical picture is used as a denomination for any array of data representing a part of a human or animal body. The pictures could be presented for the user in various ways, e.g. by means of displays and print outs. Further, the pictures could be static or dynamic. E.g. the picture could be a depiction of the status at a particular time, or be continuously updated, preferably in real time.

Usability does in this application relate to a medical usefulness of the picture, and in particular to the usefulness in diagnostic or therapeutic applications.

Entropy is in this application a denomination for a measure or a quantification of the perceptual information content of an image, and preferably a digital image. For an estimation of the entropy for this application, the information entropy estimation theory introduced by C. Shannon could be used. A greater entropy value corresponds with a greater perceptual information content in the picture.

The entropy H[X] could be described as "the uncertainty in X", not to be confused with posteriori or conditional entropy H[X/Y], which could be described as "the uncertainty in X provided knowledge of Y". The present invention uses entropy to find an intensity range for a picture, which e.g. could comprise three or more modes. This could be done by determining the entropy for a sub-range of the total intensity area. The resulting picture preferably comprises the sub-range which provides the largest entropy, or which is related to this sub-range. Thus, the entropy is calculated for a sub-range of the total intensity range for the picture, and when the sub-range is delimited, the resolution is increased within this sub-range, resulting in an increased uncertainty/entropy (thus providing more information to the observer of the output picture). At the same time, for the picture content falling outside this sub-range the uncertainty/entropy will be decreased. Thus, the entropy is used to balance these two effects.

The input medical picture is preferably generated by at least one of computed tomography (CT), magnetic resonance imaging (MRI), angiographic imaging, x-ray imaging, positron emission tomography (PET), single photon emission computerized tomography (SPECT), functional magnetic resonance imaging (fMRI) and ultrasonic imaging.

The at least one intensity parameter to be automatically controlled is at least one of brightness and contrast, and most preferably both.

Further, the intensity parameter(s) to be automatically controlled is controlled in order to reduce the gray scale window of said array of intensity data. For example, the grey scale window of a CT image may be controlled to a range of less than 500 Hounsfield, and preferably less than 250 Hounsfield, and most preferably about 100 Hounsfield.

The functional result of reducing the grey scale is to increase the dynamic range of a picture area. Consequently, a picture area with very limited color variations, e.g. varying within a range from dark grey to light grey, will, as the grey scale is reduced, be expanded into a much larger dynamic range, varying e.g. between total black and total white, and every nuance there between.

However, the intensity parameters to be controlled are parameters that affect the intensity data values in the array of intensity data. Said intensity parameters affect the brightness and/or the contrast directly or indirectly, e.g. by controlling the gray scale window.

Hounsfield is in this application used as a denomination of a normalized index of radiation (e.g. x-ray) attenuation, based on a scale of about -1000 (air) to about +1000 (bone), with water being 0, used in particular for CT imaging. The entropy of said at least a part of the array of intensity data is optimized by maximizing the entropy E, i.e. max [E], the entropy being preferably estimated as:

$$E = -\sum_{i=1}^{N} H_I(\mathbf{w})_i \log H_I(\mathbf{w})_i$$

wherein $H_I(\mathbf{w})_{1..N}$ for 1..N is the histogram of a picture $I(\mathbf{x})$ computed for the intensity parameters w, and N is the number of bins in the histogram.

The above-defined estimate of the entropy corresponds to the Shannon concept of entropy. However, other entropy concepts may be used as well. E.g. the Rényi entropy concept may be used, whereby the entropy E could be estimated as:

$$E_\alpha = \frac{1}{1-\alpha} \ln \sum_{i=1}^{N} H^\alpha(\mathbf{w})_i$$

Another possibility is to use the Havrda-Charvat concept of entropy, based on which the entropy in the present invention could be estimated as:

$$E_\alpha = \frac{1}{1-\alpha} \left( \sum_{i=1}^{N} H^\alpha(\mathbf{w})_i - 1 \right)$$

Preferably, N in the entropy definitions above is chosen to be the number of gray scale values required for the improved medical picture. This may e.g. be the number of grey scale values possible to reproduce on a certain media, such as on a certain display or printer to be used, or the number of gray scale values discernible with the human eye.

In the estimations of entropy above, **w** is preferably defined as having an upper value (upper) and lower value (lower), and wherein values $I(\mathbf{x})$ < lower are assigned to bin $H_1(\mathbf{w})_1$ and values $I(x)$ > upper are assigned to bin $H_N(\mathbf{w})_N$.

Preferably, the step of automatically controlling at least one intensity parameter is adapted to increase the entropy of a part of the array of intensity data corresponding to a certain subset of the depicted objects, and preferably corresponding to depicted structures of interest, such as soft tissue. The intensity resolution of such selected, and medically important parts is increased. What parts to be selected could be made either automatically, or controlled manually. However, it is also possible to control the intensity parameters in order to increase the overall entropy of the picture.

According to another aspect of the invention, it relates to a system for automatically improving the usability of a medical picture, comprising: input means for providing a medical picture, comprising an array of intensity data; the system characterized in that it further comprises: means for automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby providing a decreased entropy outside the sub-range; and output means for providing the processed array of intensity data of said sub-range as the improved medical picture. With this aspect of the invention, similar advantages as discussed above in relation to the first aspect are provided.

According to another aspect of the invention, it relates to a computer program for automatically improving the usability of a medical picture, comprising computer code for executing the step: providing as an input a medical picture, comprising an array of intensity data; the computer program characterized in that it further comprises computer code for executing the additional steps: automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby thereby providing a decreased entropy outside the sub-range; and providing as the processed array of intensity data of said sub-range as the improved medical picture. According to still another aspect of the invention, it relates to a data carrier comprising the computer program discussed above.

With these aspects of the invention, similar advantages as discussed above in relation to the first aspect are provided.

The invention also relates to a use of the above-discussed method for preparing medical pictures for various applications, e.g. for image processing, such as image merging or co-registration, segmentation or pattern recognition; for automized therapy treatment planning, and preferably for planning of neurosurgical treatment; and for real time monitoring and/or control during therapy, and preferably neurosurgical therapy.

These and other aspects of the invention will be apparent from and elucidated with reference to the embodiments described hereinafter.

Brief description of the drawings

For exemplifying purposes, the invention will be described in closer detail in the following with reference to embodiments thereof illustrated in the attached drawings, wherein:

Fig 1 is a schematic overview of an embodiment of the method according to the invention;
Fig 2 is a schematic overview of a process for estimating entropy of a medical picture according to an embodiment of the invention; and

Fig 3-5 illustrates examples of pictures before and after processing according to the invention.

Description of preferred embodiments

The invention will be described in the following for exemplifying purposes in more detail by means of examples.

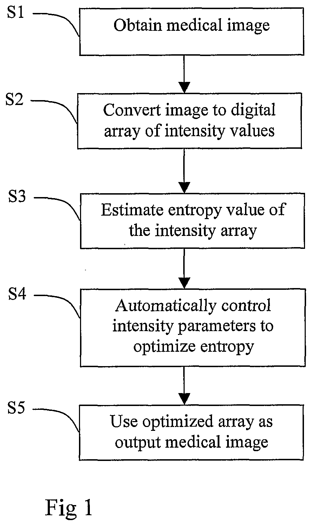

Referring first to fig 1, a method for automatically improving the usability of a medical picture according to one embodiment comprises the following steps. In a first step S1 a medical picture is input into a processing means, such as a computer, and preferably a conventional personal computer.

The medical picture is a digital image comprising an array of intensity data. However, it is also possible to use analog medical pictures, whereby an additional conversion step S2 could be used for converting the analog picture information into a digital array of intensity data, as is per se known in the art. The picture could be provided in various ways, such as with CT and MR imaging, but essentially any known medical imaging technique could be used to obtain the input image. For example, it is also feasible to provide input images by means of angiography, x-ray imaging, PET, SPECT, fMRI and ultrasonic imaging.

Thereafter, the entropy of the digital image is estimated, step S3, in a way to be discussed more thoroughly in the following.

In an optimization step S4, at least one intensity parameter is thereafter automatically controlled in order to achieve the maximum achievable entropy value under the given circumstances. The intensity parameter controlled is at least one of brightness and contrast, and most preferably both. The intensity parameters are controlled in order to reduce the gray scale window of said array of intensity data. For example, the grey scale window of a CT image may be controlled to a range of less than 500 Hounsfield, and preferably less than 250 Hounsfield, and most preferably about 100 Hounsfield.

The optimized data array is subsequently used as the improved output medical picture, step S5.

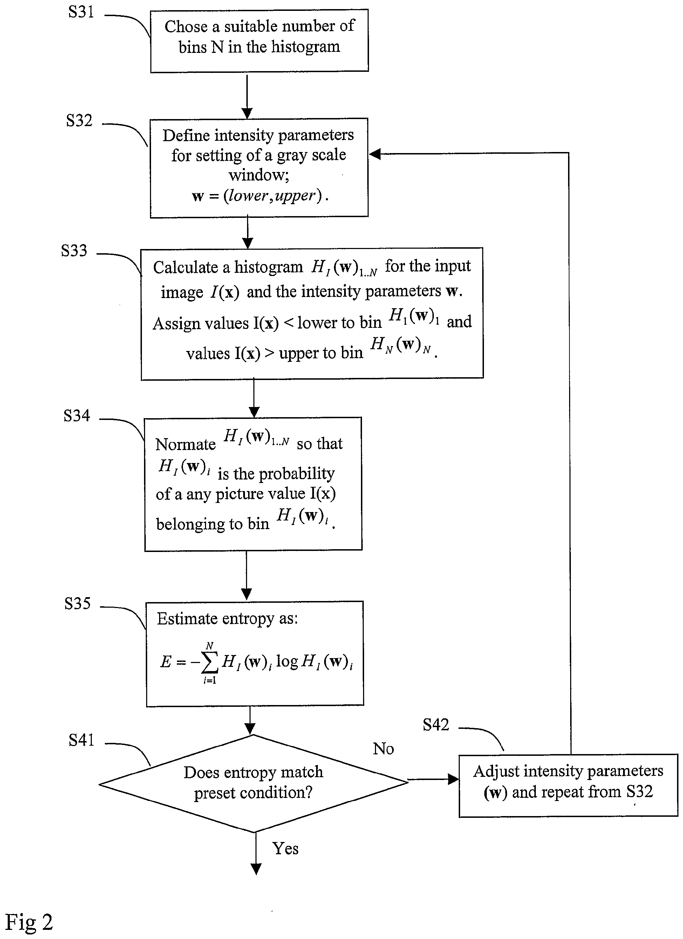

The process for estimation of the entropy and optimization of the intensity parameters will in the following be discussed more thoroughly, with reference to fig 2. Entropy is a measure of the information content in a picture or signal. Entropy is here discussed in regard of black-and-white pictures, but a similar approach may naturally be used for color pictures as well. The entropy E could be defined in various ways. E.g. the Shannon definition of entropy could be used. If so, the estimation of the entropy of a medical picture could be made in the following way:

First, the number of bins N in the histogram is chosen, step S31. Preferably N is chosen to correspond to the number of gray scale values which could be presented by the presentation means to be used, such as a display or a printer, or which the eye is capable of resolving. An initial set of intensity parameters $\mathbf{w}$ = (*lower, upper*) are defined, step S32. The intensity parameters are subsequently controlled in order to optimize the entropy, as discussed in the foregoing. A histogram $H_I(\mathbf{w})_{1..N}$ is then computed for the input image $I(\mathbf{x})$ and the intensity parameters $\mathbf{w}$, wherein values I(x) < lower are assigned to bin $H_1(\mathbf{w})_1$ and values I($\mathbf{x}$) > upper are assigned to bin $H_N(\mathbf{w})_N$ (step S33).

Then, $H_I(\mathbf{w})_{1..N}$ is normated, step S34, so that $H_I(\mathbf{w})_i$ is the probability of a any picture value I($\mathbf{x}$) belonging to that bin.

The entropy could then be calculated (step S35) as:

$$E = -\sum_{i=1}^{N} H_I(\mathbf{w})_i \log H_I(\mathbf{w})_i$$

It is then tested whether the entropy match a certain condition, such as being optimized, being at least 90% of the optimally achievable, being above a certain preset value, or the like (step S41). If not, the $\mathbf{w}$ is adjusted, and the process is repeated from step S32 (step S42).

For the automatic control of the intensity values as discussed in the foregoing, the gray scale window I($\mathbf{w}$) is optimized in order to maximize the entropy E. This optimization process could be performed in many different ways. For example, it is possible to calculate the entropy for every possible value of the intensity parameter to be controlled, or for every possible combination of values in case several parameters are controlled simultaneously, and to chose the parameter value(s) providing the highest entropy. However, it is also possible to use various optimization algorithms, such as iterative optimization methods. Several such automated optimization methods are per se known in the art. For example, it is possible to use one or several of the following optimization methods: simulated annealing, evolutionary algorithms, genetic algorithms, simplex methods, direction-set methods, conjugate gradient method and quasi-Newton methods.

The method as discussed above is implemented as a computer program comprising computer code for executing the above-related steps. The computer program could be stored on any type of data carrier, such as a RAM, ROM, CD, DVD, flash memory, etc.

The method could be executed on a data processing apparatus, such as a general purpose computer, with input means for inputting a medical picture. The input means could be a reader for extracting data from a mobile data storage, such as a disc reader, a scanner, a network connection, a port to be connected to an imaging device, or the like. Further, the apparatus is also provided with output means for providing the improved array of intensity data as the improved medical picture. The output device could comprise a display, a printer, a writer for storing data on in a data storage, and preferably a mobile data storage, such as a CD-ROM writer, or the like. The image data processing could preferably be implemented in software, but it is also possible to implement at least some part of it in especially dedicated hardware. Further, it the above-discussed parts may be contained within a single unit, or comprise distributed, interconnected parts. The method could, however, also be implemented in special equipment, such as in an ultrasonic apparatus, x-ray equipment, and the like.

The output medical pictures, resulting from the above-discussed method, may be used in various applications, and especially for different types of automatized or manual therapy and diagnostic methods. For example, the output picture could be used for controlling sterotactic surgery equipment, such as the one disclosed in WO 00/42928 by the same applicant. Hereby, the output picture could be used for measuring the destroyed volume of tissue during the lesion process. However, new method is also useful for other types of surgical procedures, and especially for neurosurgery, e.g. for real time monitoring and/or control during said therapy.

The above discussed method for improving the usability of medical pictures is also particularly useful for preparing pictures to be used in automatized therapy treatment planning, and preferably for planning of neurosurgical treatment, as discussed in WO 98/57705 by the same applicant. The improved medical pictures could also be used in other types of applications, such as for preparing pictures for other types or automated methods. E.g. said pictures could be used for preparing medical pictures for image processing, such as image merging or co-registration, segmentation or pattern recognition.

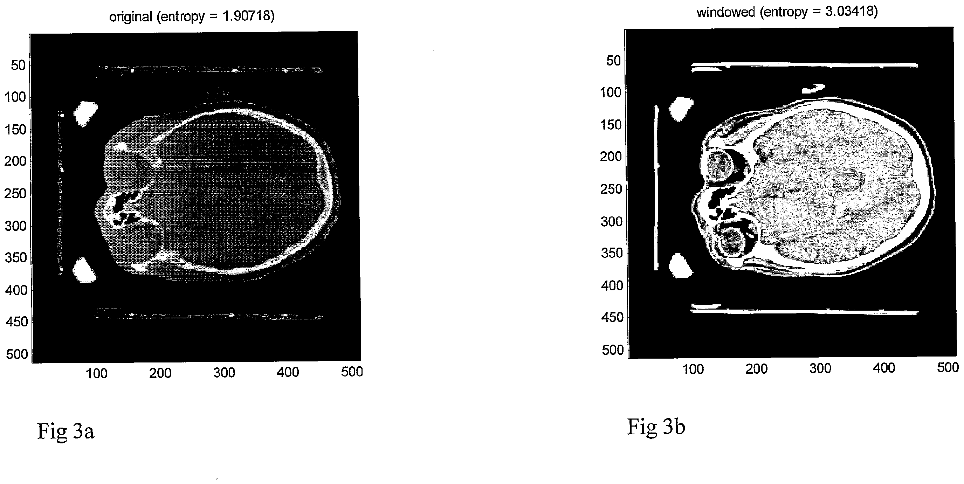

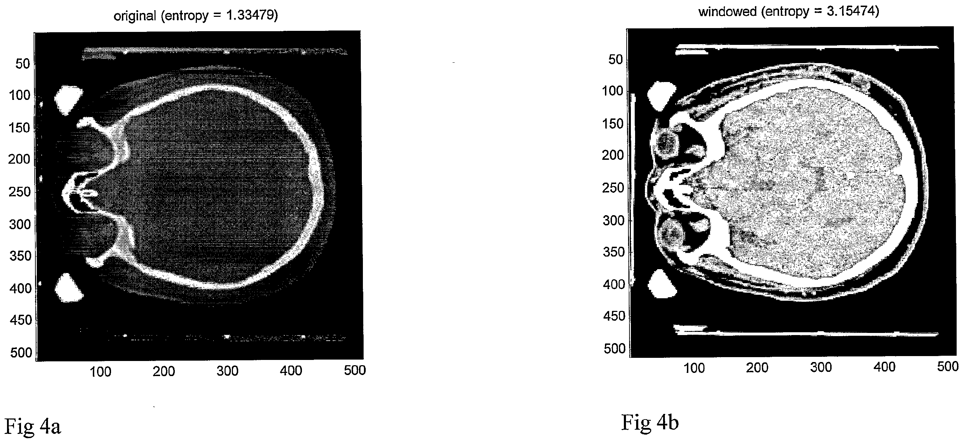

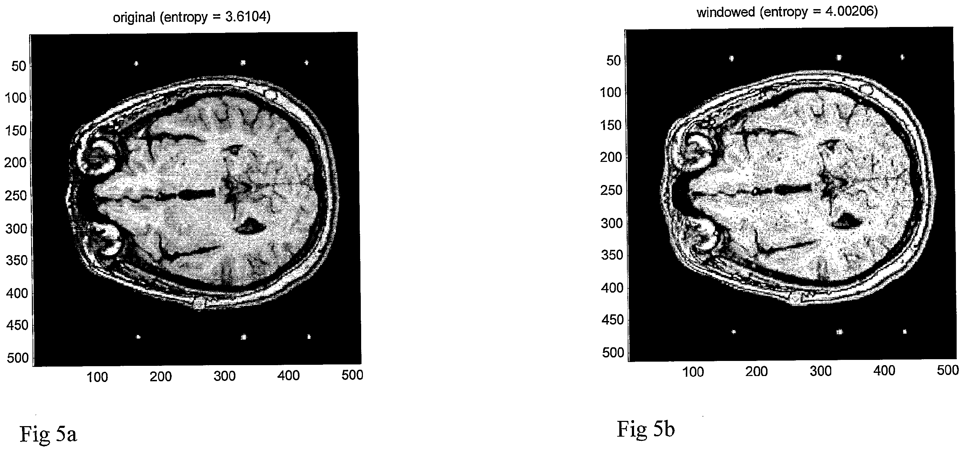

In fig 3-5 examples of the use of the present invention are provided. In fig 3a an input image is illustrated, having an original entropy of 1.907. After being processed as discussed above, an output image is obtained, as illustrated in fig 3b, having an entropy of 3.034, and consequently with a clearly enhanced visibility and intensity resolution of the soft tissue portions. Similarly, fig 4a illustrates an input image having an original entropy of 1.335, and fig 4b illustrates an improved image having an entropy of 3.155. In fig 5a, the input image has an original entropy of 3.610, whereas the improved image has an entropy of 4.002.

Specific embodiments of the invention have now been described. However, several alternatives are possible, as would be apparent for someone skilled in the art. For example, various ways to estimate entropy are feasible, the optimization could be performed in many different ways, etc.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims.

## Claims

1. Method of automatically improving the usability of a medical picture, comprising the step:
   providing as an input a medical picture, comprising an array of intensity data;
   **characterized by** the additional steps:

   automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby providing a decreased entropy outside the sub-range; and providing the processed array of intensity data of said sub-range as the improved medical picture.

2. The method of claim 1, wherein the sub-range of the intensity range is selected to a range of less than 500 Hounsfield, and preferably less than 250 Hounsfield, and most preferably about 100 Hounsfield.

3. The method of claim 1 or 2, wherein the entropy of at least a part of the array of intensity data is optimized by maximizing the entropy E, i.e. max [E], the entropy being estimated as:

$$E = -\sum_{i=1}^{N} H_I(\mathbf{w})_i \log H_I(\mathbf{w})_i$$

   wherein $H_I(\mathbf{w})_{1..N}$ is the histogram of the picture $I(\mathbf{x})$ computed for the intensity parameters w, wherein the intensity parameters are parameters that control the sub-range; and N is the number of bins in the histogram.

4. The method of claim 3, wherein N is chosen to the number of gray scale values required for the improved medical picture.

5. The method of claim 3 or 4, wherein **w** is defined as having an upper value (upper) and lower value (lower), and wherein values I(x) < lower are assigned to bin $H_1(\mathbf{w})_1$ and values I(x) > upper are assigned to bin $H_N(\mathbf{w})_N$.

**6.** The method of any one of the preceding claims, wherein the input medical picture is generated by at least one of computed tomography (CT), magnetic resonance imaging (MRI), angiographic imaging, x-ray imaging, positron emission tomography (PET), single photon emission computerized tomography (SPECT), functional magnetic resonance imaging (fMRI) and ultrasonic imaging.

**7.** The method of any one of the preceding claims, wherein the step of automatically selecting a sub-range of the intensity range is adapted to increase the entropy of a part of the array of intensity data corresponding to a certain subset of the depicted objects, and preferably corresponding to depicted structures of interest.

**8.** A system for automatically improving the usability of a medical picture, comprising:

input means for providing a medical picture, comprising an array of intensity data; the system **characterized in that** it further comprises:

means for automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby providing a decreased entropy outside the sub-range; and
output means for providing the processed array of intensity data of said sub-range as the improved medical picture.

**9.** The system of claim 8, wherein the input medical picture is generated by at least one of computed tomography (CT), magnetic resonance imaging (MRI), angiographic imaging, x-ray imaging, positron emission tomography (PET), single photon emission computerized tomography (SPECT), functional magnetic resonance imaging (fMRI) and ultrasonic imaging.

**10.** A computer program for automatically improving the usability of a medical picture, comprising computer code for executing the step:

providing as an input a medical picture, comprising an array of intensity data; the computer program **characterized in that** it further comprises computer code for executing the additional steps:

automatically selecting a sub-range of a total intensity range for the medical picture that has a maximum entropy, the intensity sub-range being directly or indirectly related to brightness or contrast, wherein the entropy is computed in such a way that it is a measure of the amount of information perceivable by the human eye, wherein the resolution in the sub-range is increased, thereby providing an increased entropy within the sub-range, and the resolution outside the sub-range is decreased, thereby providing a decreased entropy outside the sub-range; and
providing as the processed array of intensity data of said sub-range as the improved medical picture.

**11.** A data carrier for storing a computer program according to claim 10.

**12.** Use of the method according to any one of claims 1-7 for one of:

a) preparing medical pictures for image processing, such as image merging or co-registration, segmentation or pattern recognition;
b) preparing medical pictures for automized therapy treatment planning, and preferably for planning of neurosurgical treatment; and
c) preparing medical pictures for real time monitoring and/or control during therapy, and preferably neurosurgical therapy.

**Patentansprüche**

**1.** Verfahren zur automatischen Verbesserung der Nutzbarkeit eines medizinischen Bildes, umfassend den Schritt:
Bereitstellen eines medizinischen Bildes als Eingabe, umfassend ein Array von Intensitätsdaten; **gekennzeichnet durch** die zusätzlichen Schritte:

automatisches Auswählen eines Teilbereichs eines Gesamtbereichs der Intensität für das medizinische Bild, der eine maximale Entropie aufweist, wobei der Teilbereich direkt oder indirekt mit einer Helligkeit oder einem Kontrast in Beziehung steht, wobei die Entropie auf eine Weise berechnet wird, dass sie ein Maß der Menge an Informationen ist, die vom menschlichen Auge wahrnehmbar ist, wobei die Auflösung in dem Teilbereich erhöht ist, wodurch eine erhöhte Entropie innerhalb des Teilbereichs bereitgestellt wird, und die Auflösung außerhalb des Teilbereichs verringert ist, wodurch eine verringerte Entropie außerhalb des Teilbereichs bereitgestellt wird; und

Bereitstellen des verarbeiteten Arrays von Intensitätsdaten des Teilbereichs als das verbesserte medizinische Bild.

2. Verfahren nach Anspruch 1, wobei der Teilbereich des Intensitätsbereichs in einem Bereich von weniger als 500 Hounsfield-Einheiten, und vorzugsweise weniger als 250 Hounsfield-Einheiten, und am meisten bevorzugt etwa 100 Hounsfield-Einheiten ausgewählt wird.

3. Verfahren nach Anspruch 1 oder 2, wobei die Entropie von zumindest einem Teil des Arrays von Intensitätsdaten durch Maximieren der Entropie E, d. h. max[E], optimiert wird, wobei die Entropie geschätzt wird durch:

$$E = -\sum_{i=1}^{N} H_I(w)_i \log H_I(w)_i$$

wobei $\sum_{i=1}^{N} H_I(w)_{1...N}$ das Histogramm des für die Intensitätsparameter w berechneten Bildes $I(x)$ ist, wobei die Intensitätsparameter Parameter sind, die den Teilbereich steuern; und N die Anzahl der Klassen in dem Histogramm ist.

4. Verfahren nach Anspruch 3, wobei N gemäß der Anzahl von Graustufenwerten gewählt wird, die für das verbesserte medizinische Bild erforderlich sind.

5. Verfahren nach Anspruch 3 oder 4, wobei w definiert ist, einen oberen Wert (Oberwert) und einen unteren Wert (Unterwert) aufzuweisen, und wobei Werte mit $I(x) <$ Unterwert der Klasse $H_I(w)_1$ zugewiesen werden und Werte mit $I(x) >$ Oberwert (x) der Klasse $H_N(w)_N$ zugewiesen werden.

6. Verfahren nach einem der vorhergehenden Ansprüche, wobei das medizinische Eingabebild durch mindestens eines von Computertomographie (CT), Magnetresonanzbildgebung (MRI), Angiographiebildgebung, Röntgenbildgebung, Positronenemissionstomographie (PET), Einzelphotonenemissions-Computertomographie (SPECT), funktionelle Magnetresonanztomographie (fMRT) und Ultraschallbildgebung erzeugt wird.

7. Verfahren nach einem der vorhergehenden Ansprüche, wobei der Schritt des automatischen Auswählens eines Teilbereichs des Intensitätsbereichs angepasst wird, um die Entropie eines Teils des Arrays von Intensitätsdaten, der einer bestimmten Teilmenge der abgebildeten Objekte entspricht, und vorzugsweise den dargestellten Strukturen von Interesse entspricht, zu erhöhen.

8. System zur automatischen Verbesserung der Verwendbarkeit eines medizinischen Bildes, umfassend:

Eingabemittel zum Bereitstellen eines medizinischen Bildes, umfassend ein Array von Intensitätsdaten;
wobei das System **dadurch gekennzeichnet ist, dass** es ferner umfasst:

Mittel zum automatischen Auswählen eines Teilbereichs eines Gesamtbereichs der Intensität für das medizinische Bild, der eine maximale Entropie aufweist, wobei der Teilbereich direkt oder indirekt mit einer Helligkeit oder einem Kontrast in Beziehung steht, wobei die Entropie auf eine Weise berechnet wird, dass sie ein Maß der Menge an Informationen ist, die vom menschlichen Auge wahrnehmbar ist, wobei die Auflösung in dem Teilbereich erhöht ist, wodurch eine erhöhte Entropie innerhalb des Teilbereichs bereitgestellt wird, und die Auflösung außerhalb des Teilbereichs verringert ist, wodurch eine verringerte Entropie außerhalb des Teilbereichs bereitgestellt wird; und
Ausgabemittel zum Bereitstellen des verarbeiteten Arrays von Intensitätsdaten des Teilbereichs als das verbesserte medizinische Bild.

9. System nach Anspruch 8, wobei das eingegebene medizinische Bild durch mindestens eines von Computertomographie (CT), Magnetresonanzbildgebung (MRI), Angiographiebildgebung, Röntgenbildgebung, Positronenemissionstomographie (PET), Einzelphotonenemissions-Computertomographie (SPECT), funktionelle Magnetresonanztomographie (fMRT) und Ultraschallbildgebung erzeugt wird.

10. Computerprogramm zur automatischen Verbesserung der Verwendbarkeit eines medizinischen Bildes, umfassend Computercode zum Ausführen des Schrittes:

Bereitstellen eines medizinischen Bildes als Eingabe, umfassend ein Array von Intensitätsdaten;
wobei das Computerprogramm **dadurch gekennzeichnet ist, dass** es ferner Computercode zum Ausführen der zusätzlichen Schritte umfasst:

automatisches Auswählen eines Teilbereichs eines Gesamtbereichs der Intensität für das medizinische Bild, der eine maximale Entropie aufweist, wobei der Teilbereich direkt oder indirekt mit einer Helligkeit oder einem Kontrast in Beziehung steht, wobei die Entropie auf eine Weise berechnet wird, dass sie ein Maß der Menge an Informationen ist, die vom menschlichen Auge wahrnehmbar ist, wobei die Auflösung in dem Teilbereich erhöht ist, wodurch eine erhöhte Entropie innerhalb des Teilbereichs bereitgestellt wird, und die Auflösung außerhalb des Teilbereichs verringert ist, wodurch eine verringerte Entropie außerhalb des Teilbereichs bereitgestellt wird; und Bereitstellen des verarbeiteten Arrays von Intensitätsdaten des Teilbereichs als das verbesserte medizinische Bild.

11. Datenträger zum Speichern eines Computerprogramms nach Anspruch 10.

12. Verwendung des Verfahrens nach einem der Ansprüche 1 bis 7 für eines von:

a) Erstellen medizinischer Bilder zur Bildverarbeitung, wie beispielsweise Bildzusammenführung oder gemeinsame Erfassung, Segmentierung oder Mustererkennung;
b) Erstellen medizinischer Bilder zur automatisierten Therapieplanung, und vorzugsweise zur Planung einer neurochirurgischen Behandlung; und
c) Erstellen medizinischer Bildern zur Echtzeit-Überwachung und/oder Steuerung während der Therapie, und vorzugsweise der neurochirurgischen Therapie.

**Revendications**

1. Procédé d'amélioration automatique de l'aptitude à l'usage d'une image médicale, comprenant l'étape suivante :
prévision en tant qu'entrée d'une image médicale comprenant un réseau de données d'intensité ; **caractérisé par** les étapes supplémentaires suivantes :

sélection automatique d'une sous-plage d'une plage d'intensité totale pour l'image médicale qui a une entropie maximale, la sous-plage d'intensité étant directement ou indirectement en relation avec la luminosité ou le contraste, l'entropie étant calculée de manière à ce que ce soit une mesure du volume d'informations perceptible par l'oeil humain, la résolution dans la sous-plage étant accrue, en créant ainsi une entropie accrue dans la sous-plage, et la résolution à l'extérieur de la sous-plage étant réduite, en créant ainsi une entropie réduite à l'extérieur de la sous-plage ; et
fourniture du réseau traité de données d'intensité de ladite plage sous forme de l'image médicale améliorée.

2. Procédé selon la revendication 1, dans lequel la sous-plage de la plage d'intensité est sélectionnée dans une plage de moins de 500 Hounsfield, et de préférence moins de 250 Hounsfield, et le plus préférentiellement d'environ 100 Hounsfield.

3. Procédé selon la revendication 1 ou 2, dans lequel l'entropie d'au moins une partie du réseau de données d'intensité est optimisée en maximisant l'entropie E, c'est-à-dire maxi [E], l'entropie étant estimée en tant que :

$$E = -\sum_{i=1}^{N} H_I(w)_i \log H_I(w)_i$$

sachant que $H_1(w)_{1...N}$ est l'histogramme de l'image $I(x)$ calculé pour les paramètres d'intensité w, les paramètres d'intensité étant des paramètres qui contrôlent la sous-plage,
et N est le nombre de cases de l'histogramme.

4. Procédé selon la revendication 3, dans lequel N est choisi pour le nombre de valeurs d'échelle de gris requises pour l'image médicale améliorée.

5. Procédé selon la revendication 3 ou 4, dans lequel w est défini comme ayant une valeur supérieure (supérieur) est une valeur inférieure (inférieur) et des valeurs $I(x)$ < inférieur telles qu'assignées à la case $H_1(w)_1$ et des valeurs $I(x)$ > supérieur telles qu'assignées à la case $H_N(W)_N$.

6. Procédé selon l'une quelconque des revendications précédentes, dans lequel l'image médicale d'entrée est générée par au moins un procédé parmi la tomodensitométrie (TD), l'imagerie à résonance magnétique (IRM), l'imagerie angiographique, l'imagerie aux rayons X, la tomographie par émission de positrons (TEP), la tomodensitométrie par émission monophotonique (TEMP), l'imagerie à résonance magnétique fonctionnelle (IRMF) et l'imagerie ultrasonore.

7. Procédé selon l'une quelconque des revendications précédentes, dans lequel l'étape de sélection automatique d'une sous-plage de la plage d'intensité est apte à augmenter l'entropie d'une partie du réseau de données d'intensité correspondant à un certain sous-ensemble des objets représentés, et correspondant de préférence à des structures représentées d'intérêt.

8. Système d'amélioration automatique de l'aptitude à l'usage d'une image médicale, comprenant :

un moyen d'entrée pour fournir une image médicale, comprenant un réseau de données d'intensité ;
ce système étant **caractérisé en ce qu'**il comprend en outre :

un moyen de sélection automatique d'une sous-plage d'une plage d'intensité totale pour l'image médicale qui a une entropie maximale, la sous-plage d'intensité étant directement ou indirectement en relation avec la luminosité ou le contraste, l'entropie étant calculée de manière à ce que ce soit une mesure du volume d'informations perceptible par l'oeil humain, la résolution dans la sous-plage étant accrue, en créant ainsi une entropie accrue dans la sous-plage, et la résolution à l'extérieur de la sous-plage étant réduite, en créant ainsi une entropie réduite à l'extérieur de la sous-plage ; et
un moyen d'édition pour fournir le réseau traité de données d'intensité de ladite plage sous forme de l'image médicale améliorée.

9. Système selon la revendication 8, dans lequel l'image médicale d'entrée est générée par au moins un procédé parmi la tomodensitométrie (TD), l'imagerie à résonance magnétique (IRM), l'imagerie angiographique, l'imagerie aux rayons X, la tomographie par émission de positrons (TEP), la tomodensitométrie par émission monophotonique (TEMP), l'imagerie à résonance magnétique fonctionnelle (IRMF) et l'imagerie ultrasonore.

10. Programme informatique pour l'amélioration automatique de l'aptitude à l'usage d'une image médicale, comprenant un code informatique pour exécuter l'étape suivante :

prévision sous forme d'une entrée d'une image médicale comprenant un réseau de données d'un réseau de données d'intensité ;
le programme informatique étant **caractérisé en ce qu'**il comprend en outre un code informatique pour exécuter les étapes supplémentaires suivantes :

sélection automatique d'une sous-plage d'une plage d'intensité totale pour une image médicale qui a une entropie maximale, la sous-plage d'intensité étant directement ou indirectement en relation avec la luminosité ou le contraste, l'entropie étant calculée de manière à ce que ce soit une mesure du volume d'informations perceptible par l'oeil humain, la résolution dans la sous-plage étant accrue, en créant ainsi une entropie accrue dans la sous-plage, et la résolution à l'extérieur de la sous-plage étant réduite, en créant ainsi une entropie réduite à l'extérieur de la sous-plage ; et
fourniture du réseau traité de données d'intensité de ladite plage sous forme de l'image médicale améliorée.

11. Support de données pour la sauvegarde d'un programme informatique selon la revendication 10.

12. Utilisation du procédé selon l'une quelconque des revendications 1 à 7 pour une des opérations suivantes :

a) préparation d'images médicales pour le traitement d'images, comme une fusion ou un enregistrement conjoint d'images, segmentation ou reconnaissance de modèle ;
b) préparation d'images médicales pour un plan de traitement thérapeutique automatisé et de préférence pour planifier un traitement neurochirurgical ; et
c) préparation d'images médicales pour une gestion et/ou un contrôle en temps réel pendant la thérapie, et de préférence la thérapie neurochirurgicale.

S1 — Obtain medical image

S2 — Convert image to digital array of intensity values

S3 — Estimate entropy value of the intensity array

S4 — Automatically control intensity parameters to optimize entropy

S5 — Use optimized array as output medical image

Fig 1

S31 — Chose a suitable number of bins N in the histogram

S32 — Define intensity parameters for setting of a gray scale window;
$\mathbf{w} = (lower, upper)$.

S33 — Calculate a histogram $H_I(\mathbf{w})_{1..N}$ for the input image $I(\mathbf{x})$ and the intensity parameters $\mathbf{w}$. Assign values I(x) < lower to bin $H_1(\mathbf{w})_1$ and values I(x) > upper to bin $H_N(\mathbf{w})_N$.

S34 — Normate $H_I(\mathbf{w})_{1..N}$ so that $H_I(\mathbf{w})_i$ is the probability of a any picture value I(x) belonging to bin $H_I(\mathbf{w})_i$.

S35 — Estimate entropy as:
$$E = -\sum_{i=1}^{N} H_I(\mathbf{w})_i \log H_I(\mathbf{w})_i$$

S41 — Does entropy match preset condition?

No

S42 — Adjust intensity parameters (w) and repeat from S32

Yes

Fig 2

12

original (entropy = 1.90718)

Fig 3a

windowed (entropy = 3.03418)

Fig 3b

EP 1 741 058 B1

windowed (entropy = 3.15474)

Fig 4b

original (entropy = 1.33479)

Fig 4a

14

original (entropy = 3.6104)

Fig 5a

windowed (entropy = 4.00206)

Fig 5b

EP 1 741 058 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5046118 A
- US 5956435 A
- WO 0042928 A
- WO 9857705 A